(19) Europäisches Patentamt
European Patent Office
Office européen des brevets

(11) Veröffentlichungsnummer : **0 097 126**
**B1**

(12) # EUROPÄISCHE PATENTSCHRIFT

(45) Veröffentlichungstag der Patentschrift : **27.07.88**

(51) Int. Cl.⁴ : **C 07 D413/04**, A 01 N 43/82

(21) Anmeldenummer : **83810254.9**

(22) Anmeldetag : **10.06.83**

(54) **2-(3-Pyridyl)-1,3,4-oxadiazole, Verfahren zu ihrer Herstellung und ihre Verwendung in der Schädlingsbekämpfung.**

(30) Priorität : **16.06.82 CH 3724/82**

(43) Veröffentlichungstag der Anmeldung : **28.12.83 Patentblatt 83/52**

(45) Bekanntmachung des Hinweises auf die Patenterteilung : **27.07.88 Patentblatt 88/30**

(84) Benannte Vertragsstaaten : **AT BE CH DE FR GB IT LI NL**

(56) Entgegenhaltungen :
**DE-B- 1 067 439**
**US-A- 4 144 343**
**JOURNAL OF HETEROCYCLIC CHEMISTRY, Band 17, Mai 1980, Seiten 425-427, Hetero Corporation, Provo-Utah, US;G.S. PONTICELLO u.a.: "Synthesis of 2-and 6-chloro-3-(5-methyl-1,3,4-oxadiazol-2-yl)pyridines"**
**JOURNAL OF ORGANIC CHEMISTRY, Band 43, Nr. 3, 1978, Seiten 393-397, American Chemical Society, US;M. DEBELJAK-SUSTAR u.a.: "Neighboring group interaction in ortho-substituted aminopyridines. Pyridopyrimidines and related systems"**

(73) Patentinhaber : **CIBA-GEIGY AG**
**Klybeckstrasse 141**
**CH-4002 Basel (CH)**

(72) Erfinder : **Kristiansen, Odd, Dr.**
**Delligrabenstrasse 7**
**CH-4314 Möhlin (CH)**
Erfinder : **Drabek, Jozef, Dr.**
**Benkenstrasse 12**
**CH-4104 Oberwil (CH)**

**EP 0 097 126 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft 2-(3-Pyridyl)-1,3,4-oxadiazole, Verfahren zu ihrer Herstellung und ihre Verwendung in der Schädlingsbekämpfung.

Erfindungsgemäss werden neuartige 2-(3-Pyridyl)-1,3,4-oxadiazole der Formel I vorgeschlagen

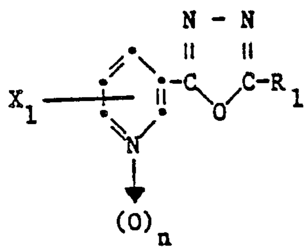

(I)

worin

$R_1$ Wasserstoff, $C_1$-$C_6$-Alkyl, $-COOR_2$ oder

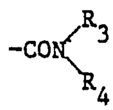

$R_2$ $C_1$-$C_6$-Alkyl, $R_3$ und $R_4$ unabhängig voneinander Wasserstoff oder $C_1$-$C_6$-Alkyl,

$X_1$ Wasserstoff, Halogen oder $C_1$-$C_6$-Alkyl und

n die Zahlen 0 oder 1 bedeuten, mit Ausnahme der Verbindungen 2-(2-Chlor-pyrid-3-yl)-5-methyl-1,3,4-oxadiazol und 2-(6-Chlorpyrid-3-yl)-5-methyl-1,3,4-oxadiazol.

Die weiterhin erfindungsgemäss zur Bekämpfung von Schädlingen verwendeten Verbindungen entsprechen der Formel I worin

$R_1$ Wasserstoff, $C_1$-$C_6$-Alkyl, $-COOR_2$ oder

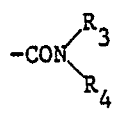

$R_2$ $C_1$-$C_6$-Alkyl, $R_3$ und $R_4$ unabhängig voneinander Wasserstoff oder $C_1$-$C_6$-Alkyl,

$X_1$ Wasserstoff, Halogen oder $C_1$-$C_6$-Alkyl und

n die Zahlen 0 oder 1 bedeuten.

Unter Halogen ist im Rahmen der Erfindung Fluor, Chlor, Brom oder Jod zu verstehen. Die Alkylgruppen bei $R_1$, $R_2$, $R_3$, $R_4$ und $X_1$ können geradkettig oder verzweigt sein. Beispiele solcher Gruppen sind u. a. : Methyl, Aethyl, Propyl, Isopropyl, n-Butyl, n-Pentyl und n-Hexyl sowie deren Isomere.

Bevorzugt werden Verbindungen der Formel I, worin

$R_1$ Wasserstoff, $C_1$-$C_3$-Alkyl, $-COOR_2$ oder

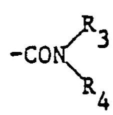

$R_2$ Methyl oder Aethyl, $R_3$ und $R_4$ unabhängig voneinander Wasserstoff, Methyl oder Aethyl,

$X_1$ Wasserstoff und

n die Zahlen 0 oder 1 bedeuten.

Die Verbindungen der Formel I können nach an sich bekannten Methoden z. B. wie folgt hergestellt werden :

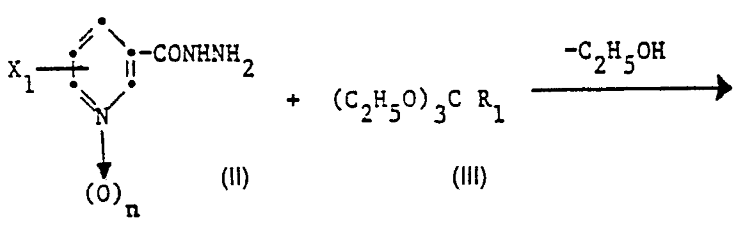


(II) (III) (I)

In den Formeln II und III haben $R_1$, $X_1$ und n die für Formel I vorstehend angegebenen Bedeutungen. Das Verfahren wird bei einer Reaktionstemperatur zwischen −50 °C und +180 °C, vorzugsweise zwischen −10 °C und +160 °C, bei normalem oder leicht erhöhtem Druck und gegebenenfalls in Gegenwart einer katalytischen Menge Säure und eines gegenüber den Reaktionsteilnehmern inerten Lösungs- oder Verdünnungsmittels durchgeführt, wobei der Orthoameisensäureester der Formel III in der Regel als Lösungsmittel genügt. Als zusätzliche Lösungs- oder Verdünnungsmittel eignen sich z. B. Aether und ätherartige Verbindungen wie Diäthyläther, Di-isopropyläther, Dioxan, Tetrahydrofuran ; aliphatische und aromatische Kohlenwasserstoffe, insbesondere Benzol, Toluol, Xylole ; Ketone wie Aceton, Methyläthylketon und Cyclohexanon.

Die Ausgangsstoffe der Formeln II und III sind bekannt bzw. können analog bekannten Methoden hergestellt werden.

In der US-Patentschrift 4 144 343 werden substituierte Pyridine beschrieben, die unter anderem substituierte Alkoxyreste als Substituenten aufweisen und als Pharmazeutica geeignet sind. Als Zwischenprodukte zur Herstellung dieser Verbindungen werden in der erwähnten US-Patentschrift bereits die Verbindungen 2-(2-Chlorpyrid-3-yl)-5-methyl-1,3,4-oxadiazol und 2-(6-Chlorpyrid-3-yl)-5-methyl-1,3,4-oxadiazol beschrieben. Demgegenüber wurde überraschenderweise gefunden, dass die erfindungsgemässen Verbindungen der Formel I ausgezeichnete Wirksamkeit als Schädlingsbekämpfungsmittel besitzen.

Die Verbindungen der Formel I eignen sich zur Bekämpfung von Schädlingen an Tieren, Pflanzen und im Boden.

Insbesondere eignen sich die Verbindungen der Formel I zur Bekämpfung von Insekten, z. B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von phytopathogenen Milben und Zecken der Ordnung Acarina.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z. B. Spodoptera littoralis und Heliothis virescens) und Gemüsekulturen (z. B. Leptinotarsa decemlineata und Myzus persicae).

In diesem Zusammenhang ist hervorzuheben, dass die genannten Verbindungen sich sowohl durch eine stark ausgeprägte systemische als auch Kontakt-Wirkung gegen saugende Insekten und vor allem gegen Insekten der Familie Aphididae (wie z. B. Aphis fabae, Aphis craccivora und Myzus persicae), welche sich mit bekannten Mitteln nur schwierig bekämpfen lassen, auszeichnen.

Wirkstoffe der Formel I zeigen auch eine sehr günstige Wirkung gegen Fliegen, wie z. B. Musca domestica und Mückenlarven. Ferner zeichnen sie sich durch eine breite ovizide und ovolarvizide Wirkung aus und besitzen eine wertvolle Wirkung gegen ektoparasitäre Milben und Zecken z. B. der Familien Ixodidae, Argasidae und Dermanyssidae.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wir Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und

Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich Alkali-, Erdalkali- oder gegebenenfalls substituierte Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im(aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgender Publikation beschrieben :

« Mc Cutcheon's Detergents and Emulsifiers Annual » MC Publishing Copr., Ringwood, New Jersey, 1982 ;

Dr. Helmut Stache « Tensid-Taschenbuch » Carl Hanser Verlag, München/Wien, 1981.

Die pestiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I (% = Gewichtsprozent)

| 1. Emulsions-Konzentrate | a) | b) | c) |
|---|---|---|---|
| Wirkstoff | 25% | 40% | 50% |
| Ca-Dodecylbenzolsulfonat | 5% | 8% | 6% |
| Ricinusöl-polyäthylenglykoläther (36 Mol AeO) | 5% | - | - |
| Tributylphenyl-polyäthylenglykoläther (30 Mol AeO) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80% | 10% | 5% | 95% |
| Aethylenglykol-monomethyläther | 20% | - | - | - |
| Polyäthylenglykol MG 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

| 3. Granulate | a) | b) |
|---|---|---|
| Wirkstoff | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| 5. Spritzpulver | a) | b) | c) |
|---|---|---|---|
| Wirkstoff | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol AeO) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

8. Extruder-Granulat

| | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulat

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1

Herstellung von 2-(3-Pyridyl)-1,3,4-oxadiazol

Das Gemisch aus 15,6 g Nikotinsäurehydrazid, 100 ml Orthoameisensäuretriäthylester und 0,1 g Kaliumhydrogensulfat wird im Oelbad auf 140 °C erwärmt. Der sich bildende Aethylalkohol lässt man abdestillieren. Nach zwei Stunden wird der überschüssige Orthoameisensäuretriäthylester im Hochvakuum abdestilliert. Nach der chromatographischen Reinigung (Kieselgel ; Eluiermittel Hexan/Toluol 1 : 1) des Rohproduktes erhält man die Verbindung Nr. 1 der Formel

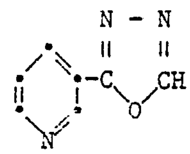

mit einem Schmelzpunkt von 78-79 °C.

Auf analoge Weise werden auch folgende Verbindungen hergestellt :

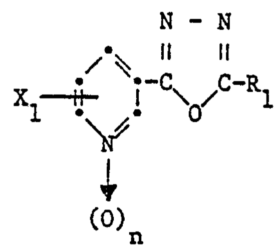

| Nr. | $R_1$ | $X_1$ | n | Physikalische Daten |
|---|---|---|---|---|
| 2 | $CH_3$ | H | 0 | 97 - 100°C |
| 3 | $C_2H_5$ | H | 0 | 40 - 41°C |
| 4 | $C_3H_7(n)$ | H | 0 | 40 - 41°C |
| 5 | H | H | 1 | 187 - 193°C |
| 6 | $-COOC_2H_5$ | H | 0 | 52-54°C |
| 7 | $-CON(CH_3)_2$ | H | 0 | $n_D^{20°}$ = 1,5822 |

Beispiel 2

Insektizide systemische Wirkung : Aphis craccivora

Bewurzelte Bohnenpflanzen werden in Töpfe, welche 60 ccm Erde enthalten, verpflanzt. Anschliessend werden 50 ml einer Versuchslösung, enthaltend 25 ppm, 5 ppm bzw. 1 ppm der zu prüfenden Verbindung, direkt auf die Erde gegossen.

Nach 24 Stunden werden auf die oberirdischen Pflanzenteile Blattläuse (Aphis craccivora) gesetzt und die Pflanzen mit einem unten zugeschnürten Plastikzylinden überstülpt, um die Läuse vor einer eventuellen Kontakt- oder Gaswirkung der Testsubstanz zu schützen.

Die Auswertung der erzielten Abtötung erfolgt 48 Stunden nach Versuchsbeginn. Pro Konzentrationsdosis Testsubstanz werden zwei Pflanzen, je eine in einem separaten Topf, verwendet. Det Versuch wird bei 25 °C und 70 % relativer Luftfeuchtigkeit durchgeführt.

Die Verbindungen gemäss Beispiel 1 haben die in der folgenden Tabelle angegebenen Wirkungen gegen Aphis craccivora.

Biologische Versuchsergebnisse

In der folgenden Tabelle sind Versuchsergebnisse auf der Basis des vorstehenden Beispiels aufgeführt, und zwar mit folgendem Bewertungsindex in Bezug auf die prozentuale Abtötung der Schädlinge :

A : 70-100 % Abtötung bei 1 ppm Wirkstoffkonzentration
B : 70-100 % Abtötung bei 5 ppm Wirkstoffkonzentration
C : 70-100 % Abtötung bei 25 ppm Wirkstoffkonzentration

| Verbindung Nr. | Wirksamkeit gegen Aphis craccivora |
|---|---|
| 1 | A |
| 2 | A |
| 3 | A |
| 4 | B |
| 5 | C |
| 6 | B |
| 7 | A |

**Patentansprüche** (für die Vertragsstaaten : BE, CH, DE, FR, GB, IT, LI, NL)

1. 2-(3-Pyridyl)-1,3,4-oxadiazole der Formel I

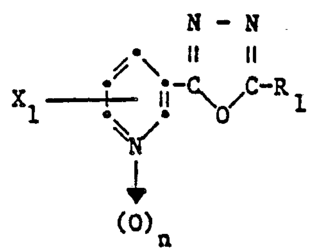

(I)

worin

$R_1$ Wasserstoff, $C_1$-$C_6$-Alkyl, —$COOR_2$ oder

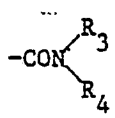

,

$R_2$ $C_1$-$C_6$-Alkyl,

$R_3$ und $R_4$ unabhängig voneinander Wasserstoff oder $C_1$-$C_6$-Alkyl,

$X_1$ Wasserstoff, Halogen oder $C_1$-$C_6$-Alkyl und

n die Zahlen 0 oder 1 bedeuten, mit Ausnahme der Verbindungen 2-(2-Chlor-pyrid-3-yl)-5-methyl-1,3,4-oxadiazol und 2-(6-Chlorpyrid-3-yl)-5-methyl-1,3,4-oxadiazol.

2. Verbindung der Formel I gemäss Anspruch 1, worin

$R_1$ Wasserstoff, $C_1$-$C_3$-Alkyl, —$COOR_2$ oder

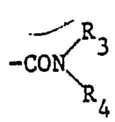

,

$R_2$ Methyl oder Aethyl,

$R_3$ und $R_4$ unabhängig voneinander Wasserstoff, Methyl oder Aethyl,

$X_1$ Wasserstoff und

n die Zahlen 0 oder 1 bedeuten.

3. Verbindung gemäss Anspruch 2 der Formel

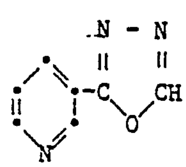

.

4. Verbindung gemäss Anspruch 2 der Formel

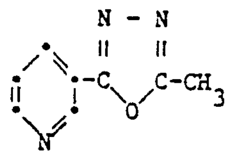

5. Verbindung gemäss Anspruch 2 der Formel

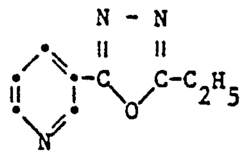 .

6. Verbindung gemäss Anspruch 2 der Formel

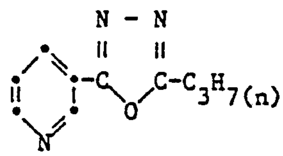

7. Verbindung gemäss Anspruch 2 der Formel

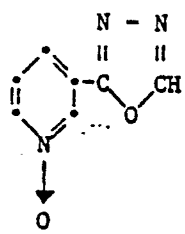

8. Verbindung gemäss Anspruch 2 der Formel

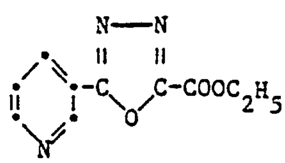 .

9. Verbindung gemäss Anspruch 2 der Formel

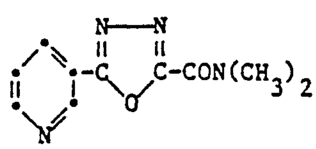

10. Verfahren zur Herstellung einer Verbindung der Formel I gemäss Anspruch 1, dadurch gekennzeichnet, dass man eine Verbindung der Formel II

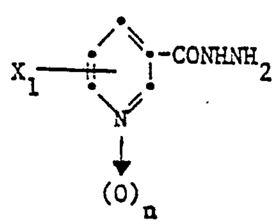

(II)

mit einer Verbindung der Formel III

$(C_2H_5O)_3CR_1$ (III)

umsetzt, wobei $R_1$, $X_1$ und n die im Anspruch 1 angegebenen Bedeutungen haben.

11. Schädlingsbekämpfungsmittel, welches als aktive Komponente eine Verbindung der Formel I gemäss Anspruch 1, enthält.

12. Verwendung einer Verbindung der Formel I gemäss Anspruch 1, worin

$R_1$ Wasserstoff, $C_1$-$C_6$-Alkyl, —$COOR_2$ oder

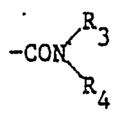,

$R_2$ $C_1$-$C_6$-Alkyl, $R_3$ und $R_4$ unabhängig voneinander Wasserstoff oder $C_1$-$C_6$-Alkyl,

$X_1$ Wasserstoff, Halogen oder $C_1$-$C_6$-Alkyl und

n die Zahlen 0 oder 1 bedeuten,

zur Bekämpfung von Insekten und Vertretern der Ordnung Akarina an Pflanzen und im Boden.

13. Verwendung gemäss Anspruch 12 zur Bekämpfung von pflanzenschädigenden, saugenden Insekten.

**Patentansprüche** (für den Vertragsstaat AT)

1. Verfahren zur Herstellung von 2-(3-Pyridyl)-1,3,4-oxadiazolen der Formel I

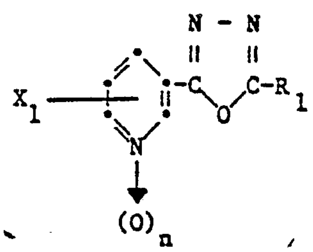

(I)

worin

$R_1$ Wasserstoff, $C_1$-$C_6$-Alkyl, —$COOR_2$ oder

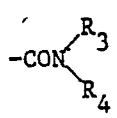,

$R_2$ $C_1$-$C_6$-Alkyl,

$R_3$ und $R_4$ unabhängig voneinander Wasserstoff oder $C_1$-$C_6$-Alkyl,

$X_1$ Wasserstoff, Halogen oder $C_1$-$C_6$-Alkyl und

n die Zahlen 0 oder 1 bedeuten, mit Ausnahme der Verbindungen 2-(2-Chlor-pyrid-3-yl)-5-methyl-1,3,4-oxadiazol und 2-(6-Chlorpyrid-3-yl)-5-methyl-1,3,4-oxadiazol, dadurch gekennzeichnet, dass man eine Verbindung der Formel II

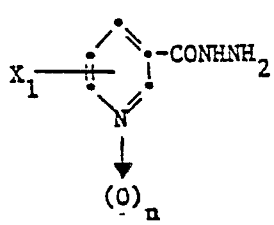

(II)

mit einer Verbindung der Formel III

$(C_2H_5O)_3CR_1$ (III)

umsetzt, wobei $R_1$, $X_1$ und n die oben angegebenen Bedeutungen haben.

2. Verfahren zur Herstellung einer Verbindung der Formel I gemäss Anspruch 1, dadurch gekennzeichnet, dass
$R_1$ Wasserstoff, $C_1$-$C_3$-Alkyl, —$COOR_2$ oder

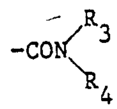 ,

$R_2$ Methyl oder Aethyl,
$R_3$ und $R_4$ unabhängig voneinander Wasserstoff, Methyl oder Aethyl,
$X_1$ Wasserstoff und
n die Zahlen 0 oder 1 bedeuten.

3. Verfahren gemäss Anspruch 2 zur Herstellung der Verbindung der Formel

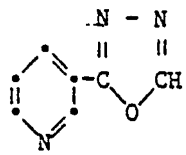

4. Verfahren gemäss Anspruch 2 zur Herstellung der Verbindung der Formel

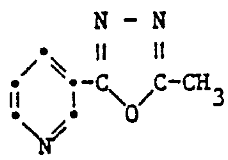 .

5. Verfahren gemäss Anspruch 2 zur Herstellung der Verbindung der Formel

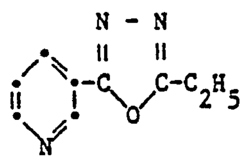 .

6. Verfahren gemäss Anspruch 2 zur Herstellung der Verbindung der Formel

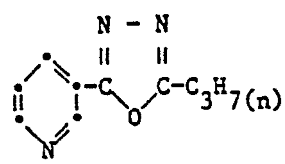 .

7. Verfahren gemäss Anspruch 2 zur Herstellung der Verbindung der Formel

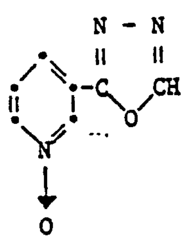

8. Verfahren gemäss Anspruch 2 zur Herstellung der Verbindung der Formel

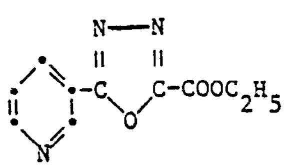 .

9. Verfahren gemäss Anspruch 2 zur Herstellung der Verbindung der Formel

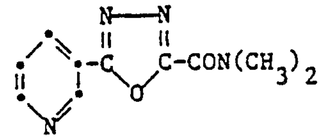

10. Schädlingsbekämpfungsmittel, welches als aktive Komponente eine Verbindung der Formel I gemäss Anspruch 1 enthält.

11. Verwendung einer Verbindung der Formel I gemäss Anspruch 1, worin

$R_1$ Wasserstoff, $C_1$-$C_6$-Alkyl, —$COOR_2$ oder

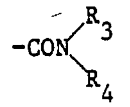 ,

$R_2$ $C_1$-$C_6$-Alkyl, $R_3$ und $R_4$ unabhängig voneinander Wasserstoff oder $C_1$-$C_6$-Alkyl,

$X_1$ Wasserstoff, Halogen oder $C_1$-$C_6$-Alkyl und

n die Zahlen 0 oder 1 bedeuten,

zur Bekämpfung von Insekten und Vertretern der Ordnung Akarina an Pflanzen und im Boden.

12. Verwendung gemäss Anspruch 11 zur Bekämpfung von pflanzenschädigenden, saugenden Insekten.

**Claims** (for the Contracting States : BE, CH, DE, FR, GB, IT, LI, NL)

1. 2-(3-pyridyl)-1,3,4-oxadiazoles of the formula I

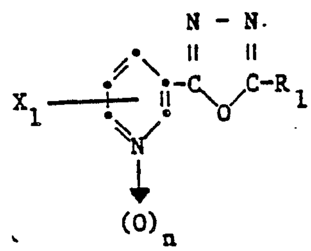

(I)

wherein

$R_1$ is hydrogen, $C_1$-$C_6$-alkyl, —$COOR_2$ or

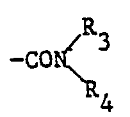 ,

$R_2$ is $C_1$-$C_6$-alkyl,

$R_3$ and $R_4$, each independently of the other, are hydrogen or $C_1$-$C_6$-alkyl,

$X_1$ is hydrogen, halogen or $C_1$-$C_6$-alkyl, and

n is 0 or 1,

with the exception of the compounds 2-(2-chloropyrid-3-yl)-5-methyl-1,3,4-oxadiazole and 2-(6-chloropyrid-3-yl)-5-methyl-1,3,4-oxadiazole.

2. A compound of the formula I according to claim 1, wherein

$R_1$ is hydrogen, $C_1$-$C_3$-alkyl, —$COOR_2$ or

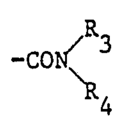 ,

$R_2$ is methyl or ethyl,

$R_3$ und $R_4$, each independently of the other, are hydrogen, methyl or ethyl,

$X_1$ is hydrogen, and

n is 0 or 1.

3. A compound according to claim 2 of the formula

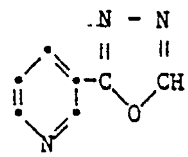

4. A compound according to claim 2 of the formula

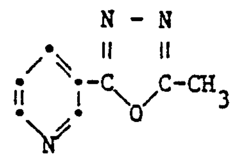
.

5. A compound according to claim 2 of the formula

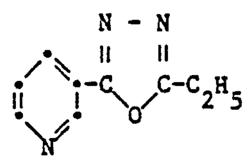
.

6. A compound according to claim 2 of the formula

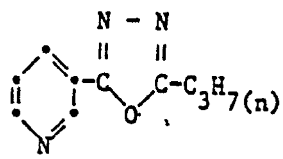

7. A compound according to claim 2 of the formula

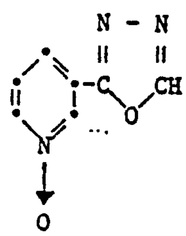

8. A compound according to claim 2 of the formula

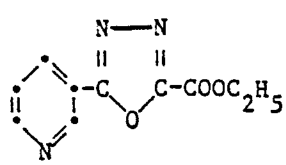
.

9. A compound according to claim 2 of the formula

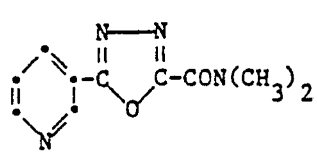

10. A process for the preparation of a compound of the formula I according to claim 1, characterised in that a compound of the formula II

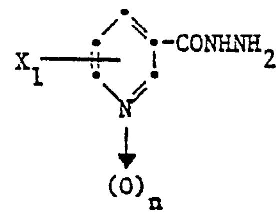

(II)

is reacted with a compound of the formula III

$$(C_2H_5O)_3CR_1 \qquad (III)$$

wherein $R_1$, $X_1$ and n are as defined in claim 1.

11. A pesticidal composition which contains a compound of the formula I according to claim 1 as active component.

12. The use of a compound of the formula I according to claim 1, wherein

$R_1$ is hydrogen, $C_1$-$C_6$-alkyl, —$COOR_2$ or

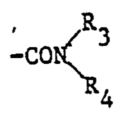

,

$R_2$ is $C_1$-$C_6$-alkyl,

$R_3$-$R_4$, each independently ot the other, are hydrogen or $C_1$-$C_6$-alkyl,

$X_1$ is hydrogen, halogen or $C_1$-$C_6$-alkyl, and

n is 0 or 1,

for controlling insects and representatives of the order Acarina on plants and in the soil.

13. The use according to claim 12 for controlling plant-destructive sucking insects.

**Claims** (for the Contracting State AT)

1. A process for the preparation of 2-(3-pyridyl)-1,3,4-oxadiazoles of the formula I

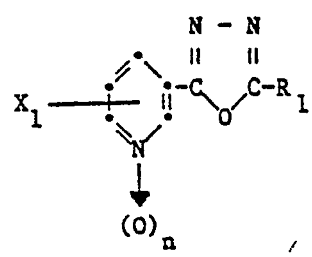

(I)

wherein

$R_1$ is hydrogen, $C_1$-$C_6$-alkyl, —$COOR_2$ or

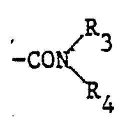

,

$R_2$ is $C_1$-$C_6$-alkyl,

$R_3$ and $R_4$, each independently of the other, are hydrogen or $C_1$-$C_6$-alkyl,

$X_1$ is hydrogen, halogen or $C_1$-$C_6$-alkyl, and

n is 0 or 1,

with the exception of the compounds 2-(2-chloropyrid-3-yl)-5-methyl-1,3,4-oxadiazole und 2-(6-chloropyrid-3-yl)-5-methyl-1,3,4-oxadiazole, characterised in that a compound of the formula II

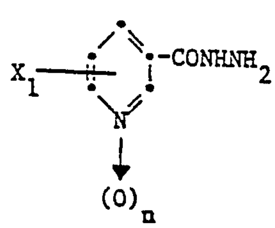

(II)

is reacted with a compound of the formula III

$$(C_2H_5O)_3CR_1 \quad \text{(III)}$$

wherein $R_1$, $X_1$ and n are as defined above

2. A process for the preparation of a compound of the formula I according to claim 1, wherein $R_1$ is hydrogen, $C_1$-$C_3$-alkyl, $-COOR_2$ or

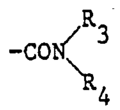 ,

$R_2$ is methyl or ethyl,
$R_3$ and $R_4$, each independently of the other, are hydrogen, methyl or ethyl,
$X_1$ is hydrogen, and
n is 0 or 1.

3. A process according to claim 2 for the preparation of a compound of the formula

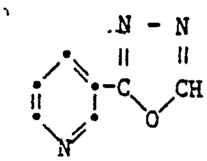

4. A process according to claim 2 for the preparation of a compound of the formula

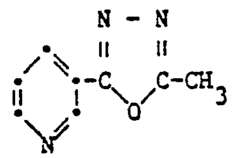 .

5. A process according to claim 2 for the preparation of a compound of the formula

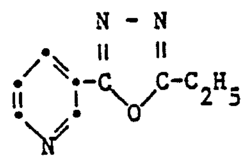

6. A process according to claim 2 for the preparation of a compound of the formula

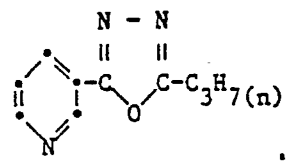 .

7. A process according to claim 2 for the preparation of a compound of the formula

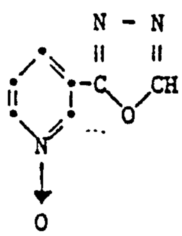 .

8. A process according to claim 2 for the preparation of a compound of the formula

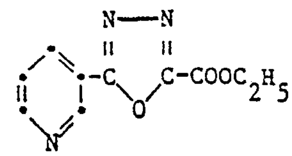

9. A process according to claim 2 for the preparation of a compound of the formula

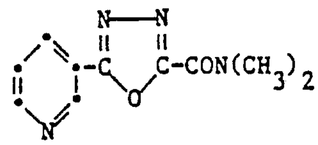

10. A pesticidal composition which contains a compound of the formula I according to claim 1 as active component.

11. The use of a compound of the formula I according to claim 1, wherein

$R_1$ is hydrogen, $C_1$-$C_6$-alkyl, —$COOR_2$ or

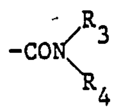

$R_2$ is $C_1$-$C_6$-alkyl,

$R_3$ und $R_4$, each independently of the other, are hydrogen or $C_1$-$C_6$-alkyl,

$X_1$ is hydrogen, halogen or $C_1$-$C_6$-alkyl, and

n is 0 or 1,

for controlling insects and representatives of the order Acarina on plants and in the soil.

12. The use according to claim 11 for controlling plant-destructive sucking insects.

**Revendications** (pour les Etats contractants : BE, CH, DE, FR, GB, IT, LI, NL)

1. 2-(3-pyridyl)-1,3,4-oxadiazoles de formule I :

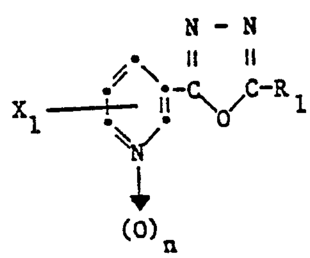

(I)

dans laquelle :

$R_1$ représente l'hydrogène, un groupe alkyle en $C_1$-$C_6$, —$COOR_2$ ou

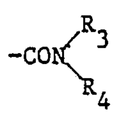

$R_2$ représente un groupe alkyle en $C_1$-$C_6$,

$R_3$ et $R_4$ représentent chacun, indépendamment l'un de l'autre, l'hydrogène ou un groupe alkyle en $C_1$-$C_6$,

$X_1$ représente l'hydrogène, un halogène ou un groupe alkyle en $C_1$-$C_6$ et

n est égal à 0 ou 1, à l'exception des composés 2-(2-chloropyrido-3-yl)-5-méthyl-1,3,4-oxadiazole et 2-(6-chloropyrido-3-yl)-5-méthyl-1,3,4-oxadiazole.

2. Composé de formule I selon la revendication 1, dans laquelle

$R_1$ représente l'hydrogène, un groupe alkyle en $C_1$-$C_3$, —$COOR_2$ ou

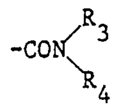 ,

$R_2$ représente un groupe méthyle ou éthyle,

$R_3$ et $R_4$ représentent chacun, indépendamment l'un de l'autre, l'hydrogène, un groupe méthyle ou éthyle,

$X_1$ représente l'hydrogène et

n est égal à 0 ou 1.

3. Composé selon la revendication 2, de formule :

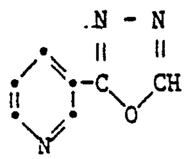 .

4. Composé selon la revendication 2, de formule :

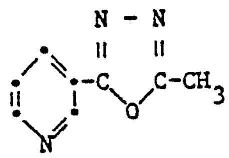 .

5. Composé selon la revendication 2, de formule :

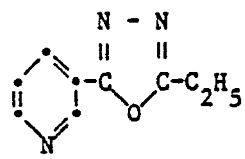 .

6. Composé selon la revendication 2, de formule :

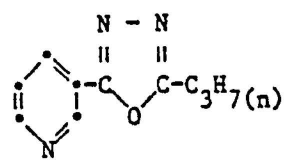

7. Composé selon la revendication 2, de formule :

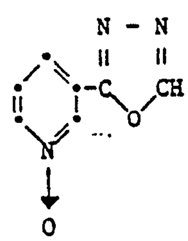 .

8. Composé selon la revendication 2, de formule :

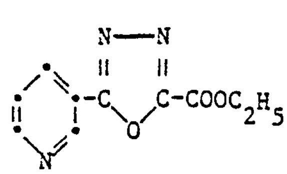 .

9. Composé selon la revendication 2, de formule :

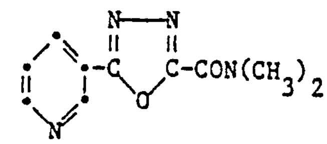 .

10. Procédé de préparation d'un composé de formule I selon la revendication 1, caractérisé en ce que l'on fait réagir un composé de formule II :

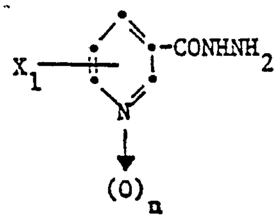

avec un composé de formule III :

$$(C_2H_5O)_3CR_1 \quad \text{(III)}$$

$R_1$ et $X_1$ et n ayant les significations indiquées dans la revendication 1.

11. Produit pesticide contenant en tant que composant actif un composé de formule I selon la revendication 1.

12. Utilisation d'un composé de formule I selon la revendication 1, dans laquelle :

$R_1$ représente l'hydrogène, un groupe alkyle en $C_1$-$C_6$, $—COOR_2$ ou

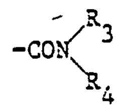 ,

$R_2$ représente un groupe alkyle en $C_1$-$C_6$,

$R_3$ et $R_4$ représentent chacun indépendamment l'un de l'autre, l'hydrogène ou un groupe alkyle en $C_1$-$C_6$,

$X_1$ représente l'hydrogène, un halogène ou un groupe alkyle en $C_1$-$C_6$ et

n est égal à 0 ou 1,

dans la lutte contre les insectes et les représentants de l'ordre des acariens sur les végétaux et dans le sol.

13. Utilisation selon la revendication 12 dans la lutte contre les insectes suceurs nuisibles pour les végétaux.

**Revendications** (pour l'Etat contractant AT)

1. Procédé de préparation des 2-(3-pyridyl)-1,3,4-oxadiazoles de formule I :

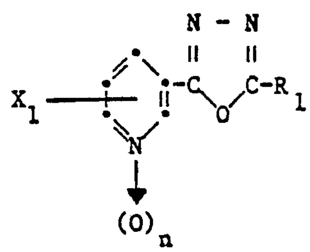

(I)

dans laquelle :

$R_1$ représente l'hydrogène, un groupe alkyle en $C_1$-$C_6$, $—COOR_2$ ou

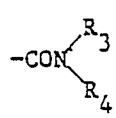 ,

$R_2$ représente un groupe alkyle en $C_1$-$C_6$,

$R_3$ et $R_4$ représentent chacun, indépendamment l'un de l'autre, l'hydrogène ou un groupe alkyle en

$C_1$-$C_6$,

$X_1$ représente l'hydrogène, un halogène ou un groupe alkyle en $C_1$-$C_6$ et

n est égal à 0 ou 1, à l'exception des composés 2-(2-chloropyrido-3-yl)-5-méthyl-1,3,4-oxadiazole et 2-(6-chloropyrido-3-yl)-5-méthyl-1,3,4-oxadiazole,

caractérisé en ce que l'on fait réagir un composé de formule II :

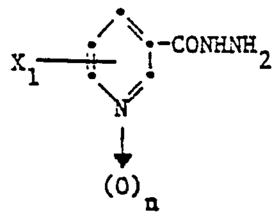

(II)

avec un composé de formule III :

$$(C_2H_5O)_3CR \qquad (III)$$

dans lesquels $R_1$, $X_1$ et n ont les significations indiquées ci-dessus.

2. Procédé de préparation d'un composé de formule I selon la revendication 1, caractérisé en ce que :

$R_1$ représente l'hydrogène, un groupe alkyle en $C_1$-$C_3$, $—COOR_2$ ou

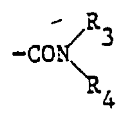,

$R_2$ représente un groupe méthyle ou éthyle,

$R_3$ et $R_4$ représentent chacun, indépendamment l'un de l'autre, l'hydrogène, un groupe méthyle ou éthyle,

$X_1$ représente l'hydrogène et

n est égal à 0 ou 1.

3. Procédé selon la revendication 2, pour la préparation du composé de formule :

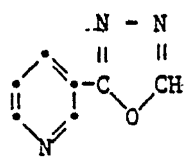.

4. Procédé selon la revendication 2 pour la préparation du composé de formule :

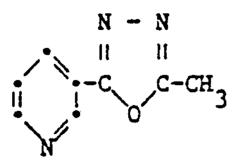.

5. Procédé selon la revendication 2 pour la préparation du composé de formule :

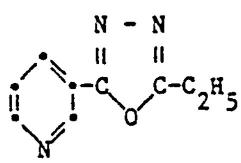.

6. Procédé selon la revendication 2 pour la préparation du composé de formule :

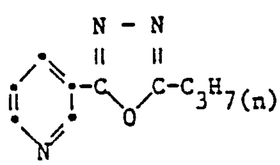.

7. Procédé selon la revendication 2 pour la préparation du composé de formule :

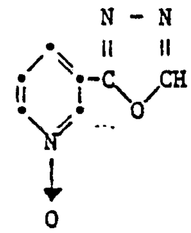
.

8. Procédé selon la revendication 2 pour la préparation du composé de formule :

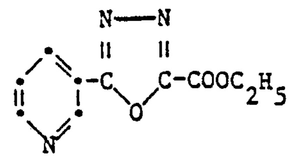
.

9. Procédé selon la revendication 2 pour la préparation du composé de formule :

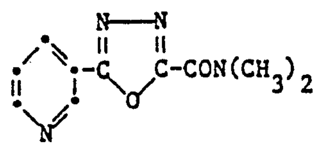
.

10. Produit pesticide contenant en tant que composant actif un composant de formule I selon la revendication 1.

11. Utilisation d'un composé de formule I selon la revendication 1 dans laquelle :

$R_1$ représente l'hydrogène, un goupe alkyle en $C_1$-$C_6$, $-COOR_2$ ou

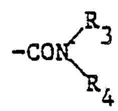
,

$R_2$ représente un groupe alkyle en $C_1$-$C_6$,

$R_3$ et $R_4$ représentent chacun, indépendamment l'un de l'autre, l'hydrogène ou un groupe alkyle en $C_1$-$C_6$,

$X_1$ représente l'hydrogène, un halogène ou groupe alkyle en $C_1$-$C_6$ et

n est égal à 0 ou 1,

dans la lutte contre les insectes et les représentants de l'ordre des acariens sur les végétaux et dans le sol.

12. Utilisation selon la revendication 11 dans la lutte contre les insectes suceurs nuisibles pour les végétaux.